# EUROPEAN PATENT SPECIFICATION

(11) **EP 0 627 198 B1**
(45) Date of publication and mention of the grant of the patent: **07.01.1999**
(21) Application number: 94200572.9
(22) Date of filing: 07.03.1994
(51) Int. Cl.: A61B 6/00

(54) **X-ray examination apparatus**
Röntgen-Untersuchungsgerät
Appareil pour examen radiologique

(30) Priority: 12.03.1993 EP 93200739
(43) Date of publication of application: 07.12.1994
(73) Proprietor: Koninklijke Philips Electronics N.V., 5621 BA Eindhoven (NL)
(72) Inventor: Hoornaert, Bart Pierre Antoine Jozef, NL-5656 AA Eindhoven (NL); Van Benthem, Adrianus Cornelis, NL-5656 AA Eindhoven (NL)
(74) Representative: Schouten, Marcus Maria

(56) References cited:
- EP-A- 0 144 670
- EP-A- 0 343 600
- EP-A- 0 496 438

## Description

The invention relates to an x-ray apparatus comprising a carrier supporting an x-ray source for producing an x-ray beam, an x-ray detector facing the x-ray source and an adjustable absorption filter which is arranged between the x-ray source and the x-ray detector, the carrier being movable to direct the x-ray beam path.

An x-ray apparatus of said kind is known from the European patent application **EP 0 496 438**.

An x-ray apparatus as described in the cited European patent application is provided with absorption filters having the form of movable wedge filters. To prevent areas in an x-ray image from being overexposed, wedge filters are positioned so that very low x-ray absorption areas adjacent to strong x-ray absorption areas are blocked out to some extent. Wedge filters are positioned in the known x-ray examination apparatus on the basis of contour recognition during the formation of the x-ray image. Consequently, complicated image processing means are required.

It is an object of the invention to provide an x-ray examination apparatus for producing an x-ray image in which overexposed areas are reduced. It is also an object of the invention to reduce a dose of x-radiation required for producing the x-ray image.

To achieve this, an x-ray examination apparatus according to the invention is characterized in that the x-ray examination apparatus is provided with filter-control means for accepting a posture of the carrier and furnishing a position of the absorption filter so as to control the adjustment of the absorption filter.

When a shadow-image is made of a part of a patient's body to be examined by means of x-irradiation, there are areas of the x-ray image in which overexposure is to be avoided in that these areas are blocked out by means of an absorption filter. The location of such areas with respect to a region of interest in the x-ray image is determined by the orientation of the x-ray beam path with respect to the patient. Since anatomical structures show a comparatively limited variation among various patients, the location of areas subjected to risk of overexposure is predominantly determined from the position of the carrier on which the x-ray source and the x-ray detector are mounted. Thus, absorption filters are adequately positioned by providing filter-control means that control the positioning of the moveable absorption filter on the basis of the posture of the carrier. In this manner, with respect to the patient and with respect to a patient support table, protracted irradiation of the patient is avoided when a person operating the x-ray examination apparatus adjusts the absorption filter. Thus, subjection of both patient on operating personnel to harmful x-radiation is reduced.

A preferred embodiment of an x-ray examination apparatus according to the invention is characterized in that the filter-control means comprises a memory device for storing pairs of position data, a pair consisting of a position data of the absorption filter and a posture of the carrier.

The filter-control means is further adequately operated by providing a memory device in which positions of the carrier and corresponding position adjustment data of the absorption filter are stored. These data constitute one or several adjustment curves which represent the positioning of the absorption filter on the basis of the posture of the carrier.

A further preferred embodiment of an x-ray examination apparatus according to the invention is characterized in that the filter-control means is arranged to furnish a translation of the absorption filter with respect to the x-ray beam path from said posture of the carrier.

In particular, overexposure is avoided in an area in the x-ray image in that the absorption filter is moved into the x-ray beam to some extent by translating the absorption filter with respect to the x-ray beam path. In this manner part of the x-ray beam is attenuated to some extent.

When the filter-control means is provided with a memory device, then the memory device will preferably contain an adjustment curve representing translation distances of the absorption filter corresponding to postures of the carrier.

A further preferred embodiment of an x-ray examination apparatus according to the invention is characterized in that the absorption filter is rotatable and comprises an x-ray absorbing part and an x-ray transmitting part and that the filter-control means is arranged to furnish an orientation of the absorption filter with respect to an axis of rotation of the absorption filter.

A sophisticated absorption filter comprises an x-ray absorbing part and an x-ray transmitting part which has the shape of a section of a substantially circular disk and such an absorption filter is slidable into or out of the x-ray beam path and is in addition rotatable about an axis of rotation making right angles to the plane of the disk section. By rotating of the absorption filter being within in or close to the x-ray beam path, portions of the x-ray beam having a comparatively more complicated form can be blocked out. In an x-ray examination apparatus according to the invention, the absorption filter is controlled by the filter control means so as to adequately position the absorption filter and block out a relevant portion of the x-ray beam. When the filter-control means is provided with a memory device, this memory device will preferably contain an adjustment curve representing orientation angles of the absorption filter corresponding to postures of the carrier.

A further preferred embodiment of an x-ray examination apparatus according to the invention is characterized in that the memory device is arranged to store a plurality of sets of said pairs of position data, each set corresponding to a class of objects to be examined.

Although the anatomical structure of various patients may be comparatively similar, there is some variation between various groups of patients, such as e.g. corpulent or slender patients or adults or infants. When patients from either group are examined, an even more adequate adjustment of the absorption filter is achieved when various adjustment curves are provided in said memory device, each adjustment curve pertaining to one of said groups of patients.

These and other aspects of the invention will become apparent from and will be elucidated with reference to the embodiments described hereinafter and with reference to the accompanying drawings.
Figure 1 shows a schematic diagram of an x-ray apparatus according to the invention.
Figure 2 shows a side elevation of a filter-arrangement for use in an x-ray apparatus according to the invention.
Figure 3 shows a top view of an absorption filter incorporated in a filter-arrangement shown in Figure 2.
Figure 4a shows a graph of a first adjustment curve, representing a rotation angle of the absorption filter as a function of beam-path orientation.
Figure 4b shows a graph of a second adjustment curve, presenting translation of the absorption filter with respect to the beam-path as a function of beam-path orientation.

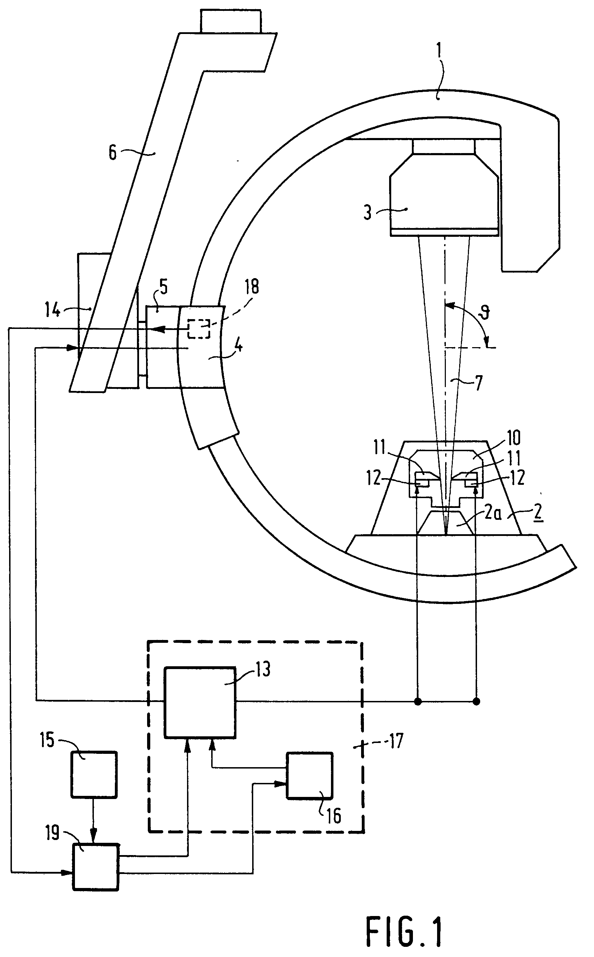

Figure 1 shows a schematic diagram of an x-ray apparatus according to the invention. A carrier 1 in the form of a C-shaped arm carrier supports an x-ray source unit 2 and an x-ray detector 3 in the form of an x-ray image intensifier facing the x-ray source unit. The carrier is moveable through a sleeve 4 and by means of a bearing 5. The carrier is attached by way of the bearing 5 to a predominantly vertical stand 6 which is rotatably mounted to a ceiling of a room in which the x-ray apparatus is set up. Thus, the x-ray source unit and the x-ray detector are moveable so as to orient a beam-path 7 of x-radiation emitted by the x-ray source unit so that various projections can be applied for making an x-ray image. A filter-arrangement 10 comprising absorption filters 11 and filter-drive-means 12 is incorporated in the x-ray source unit 2, the filter-arrangement 10 being positioned between the x-ray source 2a and the x-ray detector 3. When an x-ray image is being made, overexposure in certain areas of the x-ray image is to be avoided. Overexposure is liable to occur when regions of very low x-ray absorption and adjacent regions of high x-ray absorption are imaged in the same x-ray image. For instance, when imaging a patient's heart having a high x-radiation absorption, the surrounding lung-tissue having a low x-ray absorption will be overexposed. By covering regions of high x-ray transmittance by positioning absorption filters 11 in the x-ray beam path 7 overexposure by regions of high transmittance are avoided.

Correct positioning of the absorption filters depends on the orientation of the x-ray beam-path with respect to an object that is being examined, because the object is imaged according to a projection corresponding to the orientation of the beam-path. Filter-drive-means 12 are provided for positioning the absorption filters 11. The filter-drive-means are connected to drive-control-means 13 to which also carrier-drive-means 14 are coupled. For orienting the beam-path 7 and adjusting the absorption filters accordingly, a selection-means 15 is provided for supplying a positioning signal to the geometry controller 19. Furthermore, a posture sensor 18 is further provided to produce a posture signal pertaining to the position of the carrier. The posture signal is supplied to the geometry-controller. The geometry-controller 19 supplies a positioning signal to the drive-control-means 13, for positioning the carrier 1 in correspondence with a required beam-path orientation. The carrier-drive means 14 is controlled by the drive-control-means 13 for displacing the carrier 1. The geometry-controller 19 also applies said positioning signal to a memory-device 16 for selecting a filter-position. To this end, one or a plurality of filter-position curves is stored in the memory-device 16; each of the filter-position curves represents filter-adjustments as a function of the position of the carrier 1. When a positioning signal is supplied to the memory-device 16, a filter-adjustment signal is supplied by the memory-means to the drive-control-means 13. In this manner, filter-control-means 17 is constituted by the drive-control-means in combination with the memory device 16. Subsequently, the drive-control-means controls the filter-drive-means 12 so as to adequately position the absorption filters 11.

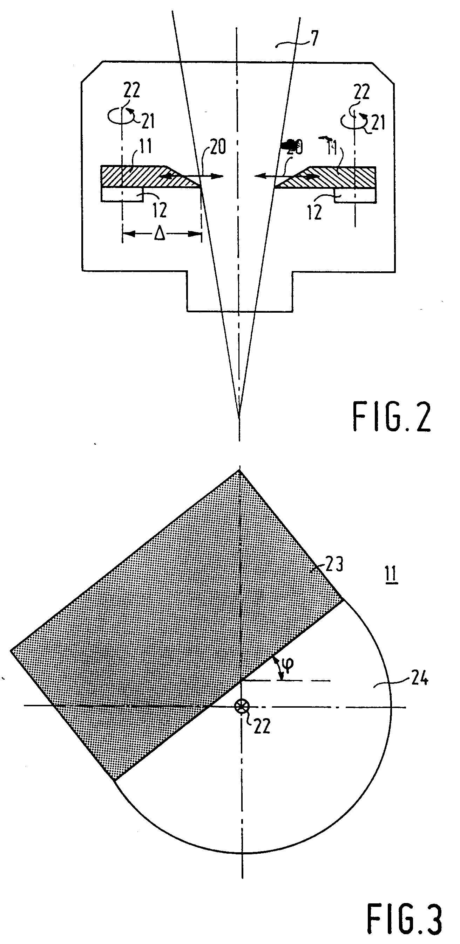

The positioning of the absorption filters will be described in more detail with reference to Figures 2 and 3. Figure 2 shows a side elevation of a filter-arrangement for use in an x-ray apparatus according to the invention. The absorption filters have a wedge-shaped extremity so that a gradual transition between a covered region and an uncovered region is obtained and consequently steep gradients in the x-ray image are avoided. The absorption filters are mounted to filter-drive-means 12 in such a way that the filters are moveable into and out of the x-ray beam path 7 as indicated by arrows 20. Furthermore the absorption filters are rotatable about axes 22 as indicated by arrows 21.

Figure 3 shows a top view of an absorption filter incorporated in a filter-arrangement shown in Figure 2. The absorption filter has an x-ray absorbing part 23, e.g. containing lead, and an x-ray transmitting part 24 which has the shape of a section of a substantially circular disk. Thus, when rotating either one of the absorption filters 11 about its axis 22 the part of the x-ray beam that impinges on the absorption filter is varied and consequently, the part of the x-ray image being covered by the absorption filter is varied.

Figure 4a shows a graph of a first adjustment curve, representing a rotation angle of the absorption filter as a function of the beam-path orientation. The rotation angle φ according to which the absorption filter is positioned is given as a function of the angle θ, which determines the orientation of the beam path with respect to a patient to be examined. For further illustration, a few particular orientations of the absorption filter have been presented as small diagrams, along the abscissa. When the carrier is positioned in accordance with θ=-80°, then the absorption filter is placed in an orientation of φ=-50°. When the carrier is moved so as to change the beam-path orientation to θ=10°, φ is increased to φ=86°. Finally, when the beam-path orientation angle is increased further, the angle of orientation of the absorption filter is slightly decreased.

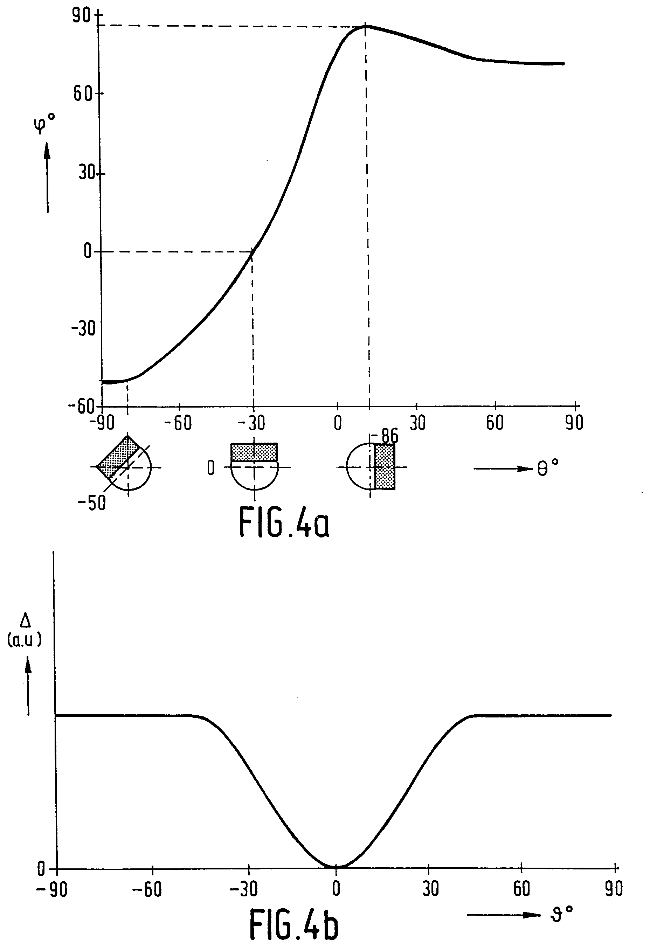

Figure 4b shows a graph of a second adjustment curve, representing translation of the absorption filter with respect to the beam-path as a function of beam-path orientation. As the curve shows, the absorption filter is moved further into the beam-path as the beam path is inclined from perpendicular incidence, viz. Δ has a minimum when θ=0° and Δ increases as ¦θ¦ increases.

The features of the adjustment curves are determined by anatomical properties, notably the location of a heart of a patient with respect to surrounding lung-tissue. In practice, these curves can be determined empirically, viz. by manually positioning absorption filters, for a multitude of carrier orientations, so as to obtain optimum image quality. It appears that a single set of adjustment curves is already quite satisfactory for a multitude of patients. Further improvement can, however, be achieved by determining sets of adjustment curves, each set pertaining to a class of patients, such as corpulent or slender patients, adults or infants, etc.

## Claims

1. An x-ray apparatus comprising
- a carrier (1) supporting an x-ray source (2) for producing an x-ray beam (7),
- an x-ray detector (3) facing the x-ray source (2) and
- an adjustable absorption filter (11) which is arranged between the x-ray source (2) and the x-ray detector (3),
- the carrier (1) being moveable to direct the x-ray beam path,
characterized in that
- the x-ray examination apparatus is provided with filter-control means (17) for
- accepting a posture of the carrier (1) and furnishing a position of the absorption filter (11) so as to control the adjustment of the absorption filter (11).

2. An x-ray examination apparatus as claimed in Claim 1, characterized in that
- the filter-control means (17) comprises a memory device (16) for storing pairs of position data,
- a pair consisting of a position data of the absorption filter (11) and a posture of the carrier (1).

3. An x-ray apparatus as claimed in any one of the preceding Claims, characterized in that
- the filter-control means (17) is arranged to furnish a translation of the absorption filter with respect to the x-ray beam path from said posture of the carrier (1).

4. An x-ray apparatus as claimed in any one of the preceding Claims, characterized in that
- the absorption filter (11) is rotatable and
- comprises an x-ray absorbing part (23) and an x-ray transmitting part (24) and that the filter-control means (17) is arranged to furnish an orientation of the absorption filter (11) with respect to an axis of rotation (22) of the absorption filter (11).

5. An x-ray examination apparatus as claimed in Claim 2, characterized in that
- the memory device is (16) arranged to store a plurality of set of said pairs of position data,
- each set corresponding to a class of objects to be examined.

## Patentansprüche

1. Röntgengerät mit
- einem Träger (1) zum Unterstützen einer Röntgenquelle (2) zum Erzeugen eines Röntgenstrahls (7),
- einem Röntgendetektor (3), der der Röntgenquelle (2) zugewandt ist, und
- einem einstellbaren Absorptionsfilter (11), der zwischen der Röntgenquelle (2) und dem Röntgendetektor (3) angeordnet ist,
- wobei der Träger (1) zum Richten des Röntgenstrahlweges verschiebbar ist,
dadurch gekennzeichnet, daß
- das Röntgenuntersuchungsgerät mit einem Filtersteuermittel (17) zum Einnehmen einer Stellung des Trägers (1) und zum Versorgen einer Position des Absorptionsfilters (11) zur Steuerung der Einstellung des Absorptionsfilters (11) versehen ist.

2. Röntgenuntersuchungsgerät nach Anspruch 1, dadurch gekennzeichnet, daß das Filtersteuermittel (17) eine Speicheranordnung (16) zum Speichern von Paaren von Positionsdaten,
und ein Paar aus Positionsdaten des Absorptionsfilters (11) und einer Stellung des Trägers (1) bestehendes Paar enthält.

3. Röntgengerät nach einem der vorangehenden Ansprüche, dadurch gekennzeichnet, daß das Filtersteuermittel (17) zum Versorgen einer Translation des Absorptionsfilters in Bezug auf den Röntgenstrahlweg aus der Stellung des Trägers (1) heraus angeordnet ist.

4. Röntgenapparat nach einem der vorangehenden Ansprüche, dadurch gekennzeichnet, daß das Absorptionsfilter (11) drehbar ist, und einen röntgenabsorbierenden Teil (23) sowie einen röntgenübertragenden Teil (24) enthält, und daß das Filtersteuermittel (17) zum Versorgen einer Orientierung des Absorptionsfilters (11) in Bezug auf eine Drehachse (22) des Absorptionsfilters (11) angeordnet ist.

5. Röntgenuntersuchungsgerät nach Anspruch 2, dadurch gekennzeichnet, daß die Speicheranordnung (16) zum Speichern einer Anzahl von Gruppen der Positionsdatenpaare angeordnet ist, wobei jede Gruppe einer Klasse zu untersuchender Objekte entspricht.

## Revendications

1. Appareil pour l'examen radiologique comprenant
- un support (1) supportant une source de rayons X (2) permettant de produire un faisceau de rayons X (7),
- un détecteur de rayons X (3) situé en face de la source de rayons X (2) et
- un filtre d'absorption réglable (11) qui est disposé entre la source de rayons X (2) et le détecteur de rayons X (3),
- le support (1) étant déplaçable afin de diriger le trajet du faisceau de rayons X,
caractérisé en ce que
- l'appareil pour l'examen radiologique est muni d'un moyen de commande de filtre (17) pour
- accepter une posture du support (1) et fournir une position du filtre d'absorption (11) de façon à commander le réglage du filtre d'absorption (11).

2. Appareil pour l'examen radiologique selon la revendication 1, caractérisé
e n ce que - le moyen de commande de filtre (17) est muni d'un dispositif de mémoire (16) pour le stockage de paires de données de position,
- une paire étant constituée par une donnée de position du filtre d'absorption (11) et une posture du support (1).

3. Appareil pour l'examen radiologique selon l'une des revendications précédentes, caractérisé en ce que
- le moyen de commande de filtre (17) est disposé de façon à fournir un déplacement de translation du filtre d'absorption par rapport au trajet du faisceau de rayons X à partir de ladite posture du support (1).

4. Appareil pour l'examen radiologique selon l'une des revendications précédentes, caractérisé en ce que
- le filtre d'absorption (11) est rotatif et
- muni d'une partie d'absorption de rayons X (23) et d'une partie de transmission de rayons X (24) et en ce que le moyen de commande de filtre (17) est disposé de façon à fournir une orientation du filtre d'absorption (11) par rapport à un axe de rotation (22) du filtre d'absorption (11).

5. Appareil pour l'examen radiologique selon la revendication 2, caractérisé en ce que
- le dispositif de mémoire (16) est disposé pour le stockage d'une pluralité de jeux desdites paires de données de position,
- chaque jeu correspondant à une classe d'objets à examiner.
